# EUROPEAN PATENT SPECIFICATION

(11) **EP 1 839 689 B1**
(45) Date of publication and mention of the grant of the patent: **26.08.2009**
(21) Application number: 07104077.8
(22) Date of filing: 13.03.2007
(51) Int. Cl.: A61M 1/00

(54) **Cassette having elastomeric clamping ribs**
Kassette mit Klemmschienen aus Elastomer
Cassette comportant des membres de fixation élastomère

(30) Priority: 29.03.2006 US 391757
(43) Date of publication of application: 03.10.2007
(73) Proprietor: Alcon, Inc., 6331 Hünenberg (CH)
(72) Inventor: Nazarifar, Nader, Laguna Niguel, CA 92677 (US); Williams, David L., Newport Beach, CA 92663 (US)
(74) Representative: Hanna, Peter William Derek

(56) References cited:
- EP-A- 1 662 142
- US-A1- 2003 135 152
- US-A1- 2003 190 244
- US-A1- 2005 230 292

## Description

### Background of the Invention

The present invention relates generally to peristaltic pumps and more specifically to fluidic cassettes used in ophthalmic surgical equipment.

Most prior art peristaltic pumps work by compressing or squeezing a length of flexible tubing (sometimes between a fixed race) using a rotating roller head. As the roller head rotates, the rollers pinch off a portion of the tubing and push any fluid trapped in the tubing between the rollers in the direction of rotation. Peristaltic pumps are widely used in medical applications because of their predictable, constant flow properties. These prior art systems, however, typically require manual connection of the pump tube segment around the rotating roller head.

Prior art peristaltic pumps using rotating roller heads also typically impart unwanted pressure pulsations. Several pulsation damping devices have been developed to address this problem (see e.g., U.S. Patent No. 4,921,477 (Davis)).

Another prior art pump is disclosed in U.S. Patent No. 6,293,926 BI (Sorensen, et al.) describes a peristaltic pump having a molded flow channel contained on an elastomeric sheet that is bonded or mechanically attached to a rigid substrate. The pump head rollers are mounted radially from the axis of rotation of the pump motor and compress the elastomeric flow channels against the rigid substrate. The commercial embodiment of the invention described in this patent is sold as the INFINITI^{®} Vision System by Alcon Laboratories, Inc., Fort Worth, Texas. This surgical console uses a Fluid Management System or cassette wherein the elastomeric sheet is friction fit into the rigid substrate without the use of any adhesives Such a construction method has proven to be extremely reliable, but this commercial system is intended primarily for cataract surgery and has no other source of vacuum other than the peristaltic pump. Therefore, the fluidic system is exposed primarily to negative pressure or vacuum and is not exposed to transient high positive pressures, such as are encountered during a reflux operation in posterior segment surgical procedures using a venturi pump as the primary source of vacuum. These high positive pressures can be as high as 700 mm Hg and have the potential to cause failure at the elastomer/substrate interface if a friction fit construction technique is used.

US 2005/230292 A (Beden et al) discloses a raised edge 52 that is part of a body 12 of its cassette, not part of a flexible elastomeric sheet (See paragraph 31, 10-12). US 2003/190244 A (Sherman et at) discloses a raised boss 60 that is part of substrate 22" of its cassette 18", not part of its elastomeric sheet 20 (See paragraph 26, lines 12-20), US 2003/135152 A (Kollar et al) discloses a raised lip portion 816 that is part of a passageway 810 of a valve chamber 808 of its cartridge 120, not part of its flexible back layer 804 (See paragraph 164, lines 2-13; paragraph 169; paragraph 170, lines 1-3). EP-1,662,142 (Debiotech SA) discloses a flexible membrane 2 having an external ridge 11 and an internal ridge 12 for mating with corresponding grooves 13 and 14 in a peristaltic pump body 3 (*See* column 4, lines 40-48).

A need continues to exist for a surgical cassette that reduces the potential for cassette failure under transient positive pressures.

### Brief Summary of the Invention

The present invention improves upon prior art cassettes by providing a cassette in accordance with the claims which follow. A molded flow channel is contained on an elastomeric sheet that is bonded or mechanically attached to a rigid substrate. A surgical console contains a cassette receiving area having a pump head with rollers that are mounted radially from the axis of rotation of the pump motor and compress the elastomeric flow channels against the rigid substrate. The cassette is held in place against the pump head by a latching mechanism that draws the cassette against the pump head. The elastomeric sheet contains a raised ridge or boss integrally formed as part of the elastomeric sheet at or near the area(s) of the elastomeric sheet that is/are subject to high transient or static pressures. The ridge allows the elastomeric sheet to be firmly pressed against the cassette receiving portion of the surgical console, yet still allow the cassette to be drawn against the pump head. The firm engagement of the sheet ridge against the cassette receiving portion of the surgical console helps to ensure that the elastomeric sheet does not become dislodged when the cassette is exposed to high transient pressures.

One objective of the present invention is to provide a peristaltic pump that uses molded elastomeric flow channels. Another objective of the present invention is to provide a peristaltic pump having radially oriented pump rollers. Yet another objective of the present invention is to provide a peristaltic pump having pump rollers that compress elastomeric flow channels against a rigid substrate. Still another objective of the present invention is to provide a cassette having an elastomeric sheet fitting to a rigid substrate.

Still another objective of the present invention is to provide a cassette having an elastomeric sheet fitting to a rigid substrate so that the elastomeric sheet does not become dislodged when the cassette is exposed to high transient pressures.

These and other advantages and objectives of the present invention will become apparent from the detailed description, drawings and claims that follow.

### Brief Description of the Drawings

FIG. 1 is a front perspective view of the cassette of the present invention.
FIG. 2 is a rear perspective view of the cassette of the present invention.
FIG. 3 is a schematic representation of the surgical cassette of the present invention installed in a surgical console. FIG. 4 is a rear perspective view of the pump elastomer sheet used on the cassette of the present invention.

### Detailed Description of the Preferred Embodiment

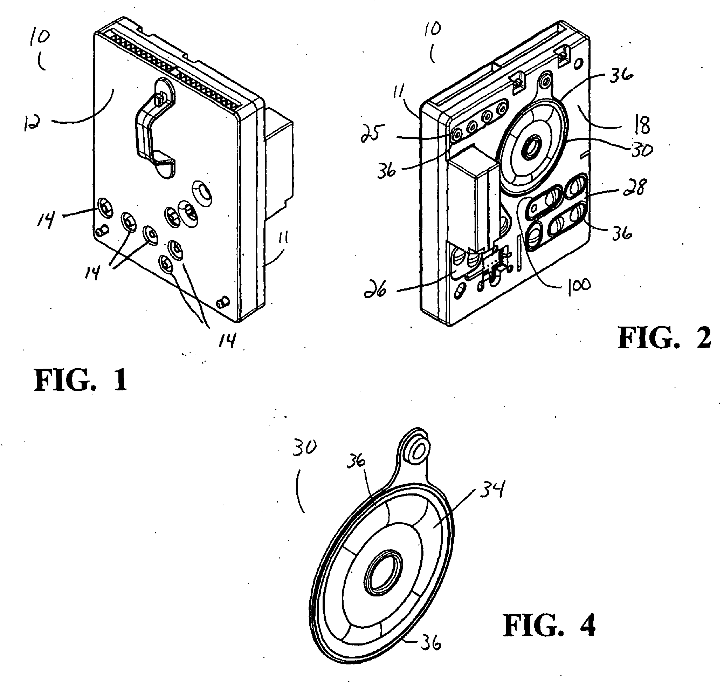

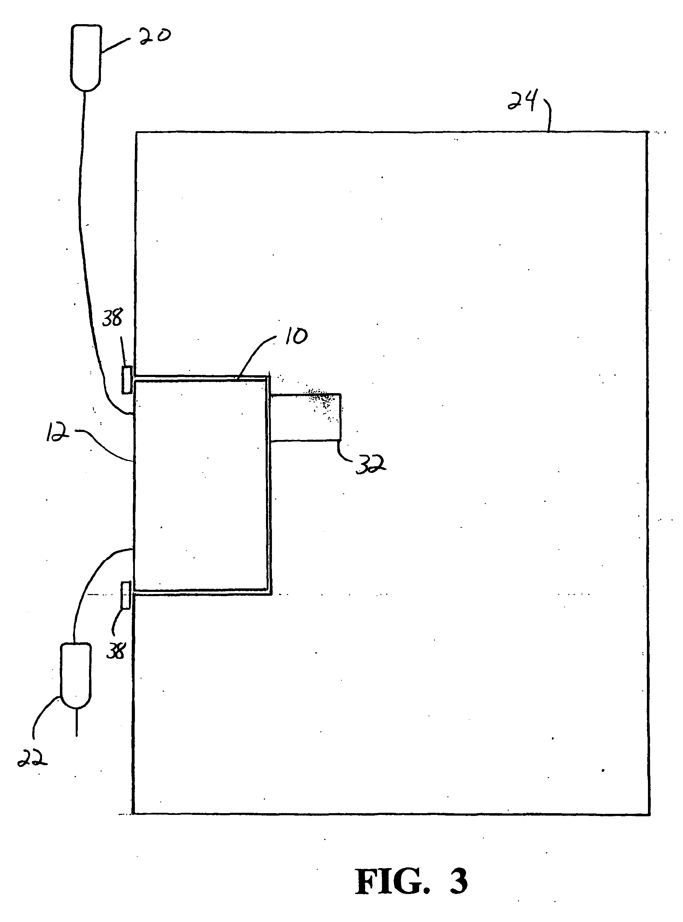

As best seen in FIGS. 1-3, cassette 10 of the present invention generally includes body 11 I having front face 12 containing a plurality of fluidic connections 14 for fluidically connecting cassette 10 to infusion fluid source 20 and surgical handpiece 22 used during a surgical procedure. Cassette 10 may also include handle 16 for assisting in installing and removing cassette 10 from surgical console 24. As best seen in FIG. 2, cassette 10 has rear face 18 opposite front face 12 and adapted for mechanical and fluidic interaction with surgical console 24. Frictionally mounted on rear face 18 of cassette 10 are a plurality of elastomeric interface-forming sheets, such as pneumatics elastomer 25, infusion elastomer 26, aspiration elastomer 28, valve elastomer 100 and pump elastomer 30. Cassette 10 is mounted within console 24 so that pump elastomer 30 is pressed against pump mechanism 32 mounted in console 24 by latches 38. Cassette 10 may be molded from any suitable material, such as thermoplastic and elastomers 25, 26, 28 and 30 may be molded from any suitable elastomer, such as silicone rubber.

As best seen in FIG. 4, pump elastomer 30 is generally circular, approximating the size and shape of the roller head (not shown) mounted to pump mechanism 32. Pump elastomer 30 contains pumping channel 34 that interacts with pump mechanism 32 to provide peristaltic pumping action through cassette 10. The operation of cassette 10 and pump mechanism 32 is more fully described in U.S. Patent No. 6,293,926 B1 (Sorensen, et al.). In order to assure that pump elastomers 25, 26, 28, 30 and 100 are firmly pressed against console 24, thereby helping to reduce the likelihood that elastomers 25, 26, 28, 30 and 100 can be dislodged from cassette 10 when exposed to high transient pressures, elastomers 25, 26, 28 and 30 contain raised ribs or bosses 36 extending around the entire areas of elastomers 25, 26, 28, 30 and 100 that are subject to high transient or static pressures. Ribs 36 are compressed when latches 38 press cassette 10 into console 24, thereby helping to prevent any movement of elastomers 25, 26, 28, 30 and 100.

This description is given for purposes of illustration and explanation. It will be apparent to those skilled in the relevant art that modifications may be made to the invention as herein described without departing from its scope as defined in the following claims.

## Claims

1. A cassette (10) adapted to be received by a surgical console (24), the cassette comprising;
a) a body (11) having a rear face (18) adapted for mechanical and fluidic interaction with the surgical console (24);
b) a relatively flexible elastomeric interface-forming sheet (30) attached to the rear face, the flexible elastomeric sheet **characterized by** at least one portion (34) which projects outwardly from the exterior of the body for defining a fluid flow channel which in use may be compressed and subjected to high transient or static fluid pressure; and
a raised rib (36), the raised rib extending around the entire area of the flexible elastomeric sheet that is subject to said high transient or static fluid pressure, the raised rib being adapted to compress against a cassette receiving portion of the surgical console.

2. The cassette of claim 1, wherein the flexible elastomeric sheet (30) is a pump elastomer sheet and the at least one portion which projects outwardly from the exterior of the body comprises at least one molded pumping channel (34),

3. The cassette of claim 1, wherein the flexible elastomeric sheet is a pneumatics elastomer sheet (25).

4. The cassette of claim 1, wherein the flexible elastomeric sheet is an aspiration elastomer sheet (28).

5. The cassette of claim 1, wherein the flexible elastomeric sheet is a valve elastomer sheet (100).

6. The cassette of claim 1, wherein the flexible elastomeric sheet is an infusion elastomer sheet (26).

## Patentansprüche

1. Kassette (10), die zur Aufnahme in einer chirurgischen Konsole (24) eingerichtet ist, wobei die Kassette aufweist:
a) einen Körper (11) mit einer Rückseite (18), die zur mechanischen und fluidischen Interaktion mit der chirurgischen Konsole (24) eingerichtet ist;
b) eine relativ flexible eine Grenzfläche bildende Elastomerfolie (30), die an der Rückseite angebracht ist, wobei die flexible Elastomerfolie **gekennzeichnet ist durch**
mindestens einen Abschnitt (34), der vom Körperäußeren aus nach außen hervorsteht, um einen Fluidströmungskanal zu definieren, der im Betrieb zusammengedrückt und mit einem hohen instationären oder stationären Fluiddruck beaufschlagt werden kann;
eine erhabene Rippe (36), wobei die erhabene Rippe um die gesamte Fläche der flexiblen Elastomerfolie, die mit dem hohen instationären oder stationären Fluiddruck beaufschlagt wird, verläuft und wobei die erhabene Rippe dazu eingerichtet ist, gegen einen Aufnahmeabschnitt für die Kassette der chirurgischen Konsole gepresst zu werden.

2. Kassette nach Anspruch 1, bei der die flexible Elastomerfolie (30) eine Pumpen-Elastomierfolie ist, und der mindestens eine Abschnitt, der vom Körperäußeren aus nach außen hervorsteht, mindestens einen geformten Pumpkanal (34) aufweist.

3. Kassette nach Anspruch 1, bei der die flexible Elastomerfolie eine Pneumatik-Elastomerfolie (25) ist.

4. Kassette nach Anspruch 1, bei der die flexible Elastomerfolie eine Ansaug-Elastomerfolie (28) ist.

5. Kassette nach Anspruch 1, bei der die flexible Elastomerfolie eine Ventil-Elastomerfolie (100) ist.

6. Kassette nach Anspruch 1, bei der die flexible Elastomerfolie eine Infusions-Elastomerfolie (28) ist.

## Revendications

1. Cassette (10) adaptée pour être reçue par une console chirurgicale (24), la cassette comprenant :
a) un corps (11) ayant une face arrière (18) adaptée pour avoir une interaction mécanique et fluidique avec la console chirurgicale (24) ;
b) une feuille de formation d'interface en élastomère relativement souple (30) fixée sur la face arrière, la feuille en élastomère souple étant **caractérisée par** au moins une partie (34) qui fait saillie vers l'extérieur à partir de l'extérieur du corps pour définir un canal d'écoulement de fluide qui, en utilisation, peut être comprimé et soumis à une pression de fluide transitoire ou statique élevée ; et une nervure surélevée (36), la nervure surélevée s'étendant autour de la surface complète de la feuille en élastomère souple qui est soumise à ladite pression de fluide transitoire ou statique élevée, la nervure surélevée étant adaptée pour être comprimée contre une partie de réception de cassette de la console chirurgicale.

2. Cassette selon la revendication 1, dans laquelle la feuille en élastomère souple (30) est une feuille en élastomère de pompe et la au moins une partie qui fait saillie vers l'extérieur à partir de l'extérieur du corps comprend au moins canal de pompage moulé (34).

3. Cassette selon la revendication 1, dans laquelle la feuille en élastomère souple est une feuille en élastomère de partie pneumatique (25).

4. Cassette selon la revendication 1, dans laquelle la feuille en élastomère souple est une feuille en élastomère d'aspiration (28).

5. Cassette selon la revendication 1, dans laquelle la feuille en élastomère souple est une feuille en élastomère de clapet (100).

6. Cassette selon la revendication 1, dans laquelle la feuille en élastomère souple est une feuille en élastomère d'infusion (26).
